(19) Europäisches Patentamt
European Patent Office
Office européen des brevets

(11) **EP 2 275 204 A1**

(12) **EUROPEAN PATENT APPLICATION**
published in accordance with Art. 153(4) EPC

(43) Date of publication:
**19.01.2011 Bulletin 2011/03**

(21) Application number: **09742662.1**

(22) Date of filing: **14.04.2009**

(51) Int Cl.:
*B01J 27/188* (2006.01)  *B01J 23/30* (2006.01)
*B01J 37/08* (2006.01)  *C07C 27/00* (2006.01)
*C07C 45/52* (2006.01)  *C07C 47/22* (2006.01)
*C07C 51/00* (2006.01)  *C07C 51/235* (2006.01)
*C07C 57/04* (2006.01)  *C07B 61/00* (2006.01)

(86) International application number:
**PCT/JP2009/057819**

(87) International publication number:
**WO 2009/136537 (12.11.2009 Gazette 2009/46)**

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(30) Priority: **16.04.2008 JP 2008107352**

(71) Applicants:
• **Nippon Kayaku Kabushiki Kaisha**
**Chiyoda-ku**
**Tokyo 102-8172 (JP)**
• **ARKEMA FRANCE**
**92700 Colombes (FR)**

(72) Inventors:
• **MAGATANI, Yasuhiro**
**SanyoOnoda-shi**
**Yamaguchi 757-8686 (JP)**
• **OKUMURA, Kimito**
**SanyoOnoda-shi**
**Yamaguchi 757-8686 (JP)**

(74) Representative: **Benson, John Everett**
**J.A. Kemp & Co.**
**14 South Square**
**Gray's Inn**
**GB-London WC1R 5JJ (GB)**

(54) **CATALYST FOR PRODUCING ACROLEIN AND ACRYLIC ACID THROUGH GLYCERIN DEHYDRATION AND PRODUCTION METHOD OF SAME**

(57)    Catalyst used in a process for preparing acrolein and acrylic acid at higher yield to convert glycerin to valuable other chemical raw materials. The glycerin dehydration catalyst consists mainly of a compound containing at least one element selected from Mo, W and V, in which protons in the heteropolyacid are exchanged at least partially with at least one cation selected from elements belonging to Group 1 to Group 16 of the Periodic Table of Elements.

EP 2 275 204 A1

**Description**

**Technical Field**

This invention relates to a novel dehydration catalyst, in particular to a dehydration catalyst for producing acrolein or acrylic acid by catalytic dehydration of glycerin in gas phase or liquid phase, and a method for producing the catalyst.

**Background Art**

Known industrial process for producing unsaturated aldehyde and unsaturated carboxylic acid such as acrolein and acrylic acid is a gas phase oxidation reaction of propylene as a starting material effected in the presence of a catalyst. However, from the viewpoint of global warming and petroleum depletion, it is requested recently to develop another technique to produce fuels and organic products from bio resources which do not depend on fossil resources. Examples of such products are bio-ethanol and bio-diesel obtained by a conversion of fats and oils, the output of which may exceed 20 million tons/year. Since they are produced from vegetable oil, they can be an alternative fuel of fossil fuel and decrease discharge of carbon dioxide, so that their increase in demand is expected.

Glycerin as a byproduct is produced in a process for preparing the bio-diesel and the quantity of glycerin is such large as about 1/10 of the total output, resulting in that a large amount of glycerin is disposed in a form of industrial waste due to in balance between demand and supply and only a part of glycerin produced is utilized as materials for medicines, cosmetic and foodstuff additives. Therefore, new synthesis reactions are researched very actively to develop novel uses of glycerin and/or to expand its demand.

The conversion process of glycerin to chemical materials with keeping its skeleton of three carbons can be classified roughly into two reactions of dehydration reaction and oxidation reaction. In this invention, the dehydration reaction will be explained with reference to acrolein production but the oxidation reaction is not explained here.

Several processes for producing acrolein were already proposed. For example, in U.S. Patent No.2,558,520, acrolein is obtained with a yield of 72% by using a catalyst of phosphoric acid supported on a carrier such as diatomaceous earth. The catalyst is dispersed in an organic solvent having a high boiling point in to which glycerin is added drop-wise to effect liquid-phase dehydration reaction.

This process, however, may not be used in industrial scale, because a large amount of carbides is produced and a separation stage for a liquid mixture comprising the carbide, catalyst and products is necessary.

JP-A1-2006-290815 discloses a process for producing acrolein in which glycerin is dispersed in a solvent and is subjected to a liquid-phase dehydration reaction in the presence of an acid solid catalyst possessing H0 of -5.6 to +3.3 such as $KHSO_4$ and $K_2SO_4$.

In this process also, more than 10% of carbides is produced, so that the process can hardly say as an industrial sufficient process and improvement is necessary.

When acrolein is produced by a liquid-phase or gas-phase contact reaction of glycerin, solid acid catalysts possessing H0 of less than +2 are effective. An example of such solid acid catalysts is phosphoric acid catalyst supported on $\alpha$-$Al_2O_3$ as is shown in JP-A1-6-211724.

In this patent, acrolein is obtained at a yield of 75% in gas-phases at 300° C but the life of catalyst is short. Still more, amounts of by-products such as hydroxy acetone increase in time and the selectivity also is not so high.

WO2007/058221 disclose processes for producing acrolein, by a dehydration reaction of glycerin in gas-phase in the presence of heteropolyacid used as a solid acid catalyst.

The heteropolyacid is those of Group 6 element such as tungstosilicic acid, tungstophosphoric acid and phosphomolybdic acid. These heteropolyacids are supported on bi-elemental pore silica carrier and produce acrolein at a yield of 86%. This dehydration reaction of the glycerin, however, is effected with free of oxidation gas but using nitrogen stream as carrier gas, so that deposition of carbon increase seriously and hence there is a problem of deterioration in time of stability, activity and selectivity of the catalysis.

Tsukuda et al., "Production of acrolein from glycerol over silica-supported heteropoly acids", CATALYSIS COMMUNICATIONS, vol. 8, no. 9, 21 July 2007, pp. 1349-1353 discloses that supported heteropoly acids were effective as a catalyst for the dehydration of glycerol.

In WO2006/087083, oxygen is introduced to prevent degradation of the catalyst in the gas-phase reaction of glycerin. InWO2006/087084, the catalyst possessing the acid strength of H0 of -9 to -18 is used.

In these patents, a variety of solid acid catalysts such as phosphoric acid/zirconia, Nafion/silica, sulfuric acid/zirconia, tungsten/zirconia are used in Examples and the highest yield of acrolein of 74% was obtained when tungsten/zirconia catalyst was used.

However, there is no catalyst usable in the industrial scale at higher performance.

## Disclosure of Invention

### Technical Problems

An object of this invention is to provide a dehydration catalyst used in a production of acrolein and acrylic acid from glycerin which is a material not derived from petroleum, at a high yield.

A specific object of this invention is to provide a catalyst used in dehydration reaction of glycerin to produce acrolein at high yield.

A further object of this invention is to provide a method for preparing the catalyst.

### Technical Solution

Inventors of this application have made a variety of studies to solve the problems and found that acrolein and acrylic acid can be produced at high yield by the dehydration reaction of glycerin by using a compound in which protons in heteropolyacid are exchanged at least partially with at least one cation selected from elements belonging to Group 1 to Group 16 of the Periodic Table of Elements, and completed this invention.

This invention has following features (1) to (8) taken separately or in combination:

(1) Dehydration catalyst for producing acrolein and acrylic acid by catalytic dehydration reaction of glycerin, containing, as a main component, at least one compound in which protons in a heteropolyacid are exchanged at least partially with at least one cation selected from elements belonging to Group 1 to Group 16 of the Periodic Table of Elements.

(2) The dehydration catalyst for producing acrolein and acrylic acid by a catalytic dehydration reaction of glycerin consisting mainly of a compound in which protons in said heteropolyacid are exchanged at least partially with at least one cation selected from elements belonging to Group 1 to Group 16 of the Periodic Table of Elements, wherein the slat of heteropolyacid is represented by the general formula (1):

$$H_a A_b [X_1 Y c Z_d O_e] \cdot nH_2O \qquad (1)$$

in which

H is hydrogen,

A is at least one cation selected from elements belonging to Group 1 to Group 16 of the Periodic Table of Elements except H,

X is P or Si,

Y is at least one element selected from the group comprising W, Mo, Ti, Zr, V, Nb, Ta, Cr, Mn, Fe, Co, Ni, Cu, Zn, Ga, In, T1, Sn and Pb,

Z is at least one element selected from the group comprising W, Mo, Ti, Zr, V, Nb, Ta, Cr, Mn, Fe, Co, Ni, Cu, Zn, Ga, In, Tl, Sn and Pb, and a, b, c and d satisfying following ranges:

$0 \leq a < 9$
$0 < b \leq 9$
$0 < c \leq 12$ and
$0 \leq d < 12$

e is a number determined by the oxidation of the elements and

n is any positive number.

(3) The cation is at least one alkali metal cation.

(4) The alkali metal is cesium.

(5) The heteropolyacid is heteropolyacid of at least one element selected from a group comprising W, Mo and V.

(6) The catalyst containing further at least one another salt of elements selected from a group comprising elements belonging to Group 1 to Group 16 of the Periodic Table of Elements, in addition to said slats of heteropolyacid.

(7) The catalyst is used in a form of a catalyst supported on a carrier.

(8) The catalyst is produced by calcination.

### Advantageous Effect

The process according to this invention in which glycerin is catalytic dehydrated to prepare acrolein and acrylic acid is very advantageous for industrial uses, because acrolein and acrylic acid can be produced at higher yield and in higher efficiency. In fact, the resistance to water is remarkably improved and deactivation of catalyst can be suppressed effectively by using the compound according to this invention. On the contrary, in case of the conventional catalyst of heteropolyacids, deterioration or deactivation of catalysts is serious in the glycerin dehydration reaction in a gas phase reaction which is effected in the presence of excess amount of water, such a reaction using as a material an aqueous solution of glycerin at lower concentration, or in a liquid phase in which water or lower alcohol is used as a reaction medium. Still more, owing to the improvement in resistance to water, a problem of corrosion of reactors that was observed when acid catalyst was used can be also solved

## Best Mode for Carrying out the Invention

The dehydration catalyst of this invention is used in a dehydration of glycerin to produce acrolein and acrylic acid and comprises mainly a compound in which protons in said heteropolyacid are exchanged at least partially with at least one cation selected from elements belonging to Group 1 to Group 16 of the Periodic Table of Elements. Generally, the dehydration catalyst of this invention has a structure of heteropolyacid.

The heteropolyacid is known and have several structures such as Keggin type, Dawson type and Anderson type and possess generally such high molecular weight as 700 to 8,500. There are dimer complex forms and those dimer complex are included in the present invention.

The elements belonging to Group 1 to Group 16 of the Periodic Table of Elements may be sodium, potassium, rubidium, cesium, magnesium, calcium, strontium, barium, scandium, yttrium, lanthanide, titanium, zirconium, hafnium, chromium, manganese, rhenium, iron, ruthenium, osmium, cobalt, nickel, palladium, platinum, copper, silver, gold, zinc, gallium, thallium, germanium, tin, lead, bismuth and tellurium. The onium salts of heteropolyacid may be amine salts, ammonium salts, phosphonium salts and sulfonium salts.

The glycerin dehydration catalyst according to the present invention is used for producing acrolein and acrylic acid. The above-mentioned compounds in which protons are exchanged at least partially with at least one cation preferably contain at least one element selected from a group comprising,W, Mo and V.

Ions of molybdenum and of tungsten form oxoacid in water and the oxoacids polymerize to form the polyoxoacid of high molecular weight. The polymerization may not be effected only with the same kind of oxoacids but also with other kinds of oxoacids. Heteropolyacid is a polyacid possessing polynuclear structure, obtained by condensation of more than two kinds of oxoacids. An atom which forms the center oxoacid is called as "hetero-atom", while atoms forming oxoacids surrounding the center oxoacid and obtained by the polymerization is called as "poly-atoms". The hetero-atom may be silicon, phosphorus, arsenic, sulfur, iron, cobalt, boron, aluminum, germanium, titanium, zirconium, cerium and chromium. Among them, phosphorus and silicon are preferable. The poly-atoms may be molybdenum, tungsten, vanadium, niobium and tantalum. Among them, molybdenum and tungsten are preferable. The heteropolyacids used in this invention to prepare a glycerin dehydration catalyst may be tungstophosphoric acid, tungstosilicic acid, phosphomolybdic acid and siloco molybdic acid. The heteropolyacid may be a mixed coordinate comprising the hetero-atoms of phosphorus or silicon and the poly-atoms are mixed coordinate of molybdenum and tungsten, or mixed coordinate of tungsten and vanadium or mixed coordinate of vanadium and molybdenum.

In a preferred embodiment, the glycerin dehydration catalyst according to this invention consists mainly of a compound in which at least part of protons in the heteropolyacid are exchanged with at least one cation of alkali metal.

When this glycerin dehydration catalyst is used, it is possible to produce acrolein and acrylic acid at high yield. In a preferred embodiment, the alkali metal is preferably cesium and at least a part of protons in the heteropolyacid is exchanged with cesium. It is also possible to exchange at least a part of protons in the heteropolyacid with cesium and a part of remaining protons in the heteropolyacid is exchanged at least partially with at least one cation selected from elements belonging to Group 1 to Group 16 of the Periodic Table of Elements. This glycerin dehydration catalyst also is effective to produce acrolein and acrylic acid at high yield. Resistance to water is increased by exchanging part of protons contained in the heteropolyacid with cesium, so that the life of catalyst is improved in comparison to heteropolyacid that is inherently water-soluble.

An amount of the aqueous solution of mineral salt of exchanging cation is determined in such a manner that an electric charge of cation to be added is equal to or less than an electric charge of the heteropolyanion. For example, when a cation with charges of 1+ is added to a heteropolyanion with charges of 3-, the cation is added equal to or less than 3 equivalent to the heteropolyanion, and when a cation with charges of 3+ is added to a heteropolyanion with charges of 3-, the cation is added equal to or less than 1 equivalent to the heteropolyanion. When a plurality of cations are introduced, an amount of the cation is determined in such a manner that the total electric charge of the cations becomes equal to or less than an electric charge of the heteropolyanion. If an amount of an aqueous solution of inorganic salt or a proportion of the cation(s) to be exchanged with protons become excessive, the activity of catalyst is spoiled or the yields of acrolein and acrylic acid are lowered or the life of catalyst is shortened.

In a variation, the glycerin dehydration catalyst according to this invention contains further at least compound of elements belonging to Group 1 to Group 16 of the Periodic Table of Element in addition to the above compound. The compound of elements belonging to Group 1 to Group 16 of the Periodic Table of Element may be metal salts or onium salts. The metal salt may be salt of tellurium, platinum, palladium, iron, zirconium, copper, cerium, silver and aluminum. The onium salts may be amine salts, ammonium salts, phosphonium salts and sulfonium salts. The metal salt or the onium salt may be prepared from such materials as nitrates, carbonate, sulfates, acetates, oxides and halides of the metals or of onium but are not limited thereto. A proportion of the metal salt is 0.01 to 60 % by weight, preferably 0.01 to 30 % by weight in term of the metal salts or the onium salt with respect to the above compound.

As the mostly preferred dehydration catalyst of glycerin, following composition represented by the general formula (1) is mentioned:

$$H_a A_b [X_1 Y c Z_d O_e] \cdot n H_2 O \qquad (1)$$

in which
H is hydrogen,
A is at least one cation selected from elements belonging to Group 1 to Group 16 of the Periodic Table of Elements except H
X is P or Si,
Y is at least one element selected from the group comprising W, Mo, Ti, Zr, V, Nb, Ta, Cr, Mn, Fe, Co, Ni, Cu, Zn, Ga, In, Tl, Sn and Pb,
Z is at least one element selected from the group comprising W, Mo, Ti, Zr, V, Nb, Ta, Cr, Mn, Fe, Co, Ni, Cu, Zn, Ga, In, T1, Sn and Pb, and a, b, c and satisfying following ranges:

$0 < a < 9$
$0 < b < 9$ '
$0 < c < 12$ and
$0 < d < 12$

e is a number determined by the oxidation of the elements and n is any positive number.

In the glycerin dehydration catalyst according to this invention, the above compound can be supported on a carrier ("supported catalyst"). Examples of the carrier are silica, diatomaceous earth, alumina, silica alumina, silica magnesia, zirconia, titania, magnesia, zeolite, silicon carbide and carbide. The catalyst can be supported on a single carrier or a complex or mixture of at least two carriers. Active material can be used effectively by supporting the active material in carrier. An amount of the heteropolyacid salt is 5 to 99.9 % by weight, preferably 5 to 90 % by weight to the weight of the carrier.

In a variation, in place of supporting the compound in which protons in a heteropolyacid are exchanged with at least one cation selected from elements belonging to Group 1 to Group 16 of the Periodic Table of Elements, it is possible to effect such operation that heteropolyacid is applied firstly onto a carrier and then the exchange with cation is carried out.

The catalyst may have any shape and can be granule or powder. In case of gas phase reactions, however, it is preferable to molded the catalyst into a shape of sphere, pellets, cylinder, hollow cylinder, bar or the like optionally with adding a molding aide or the catalyst is shaped into these configuration together with carrier and optional auxiliary agents. A size of molded catalyst is for example 1 to 10 mm for a fix bed and less than 1 mm for a fluidized bed.

The compound used in the present invention can be prepared by known technique. For example, an aqueous solution of heteropolyacid is prepared firstly. The aqueous solution of heteropolyacid may be produced after waters in heteropolyacid contained in a form of adsorptive water and crystal water are partially or perfectly removed under vacuum or heat-drying. Then, an aqueous solution of halide, carbonate, acetate, nitrate, oxalate, phosphate or sulfate of metal or onium is added to the aqueous solution of heteropolyacid. From the resulting product, a solid component is obtained by suitable treatment such as evaporation-drying, filtering and vacuum-drying. The solid component is finally fired or calcinated to obtain the catalyst for glycerin dehydration reaction according to the present invention.

The catalyst according to the present invention used in the glycerin dehydration may be anhydrides or hydrates. In fact, they can be used after pretreatment of firing and vacuum-drying or without pretreatment.

The calcination can be carried out in air or under inert gas such as nitrogen, helium and argon or under an atmosphere of mixed gas of air and inert gas usually in a furnace such as muffle furnace, rotary kiln, fluidized bed furnace. The furnace is not limited specially. The calcination can be effected even in a reaction tube which is used for the glycerin dehydration reaction. The firing temperature is usual 150 to 900°C, preferably 200 to 800°C and more preferably 200 to 600°C. The calcination is continued usually for 0.5 to 10 hours.

The dehydration reaction of glycerin according to this invention can be carried out in gas phase or in liquid phase and the gas phase is preferable. The gas phase reaction can be carried out in a variety of reactors such as fixed bed, fluidized bed, circulating fluidized bed and movable bed. Among them, the fixed bed is preferable. Generation of the catalyst can be effected outside the reactor. When the catalyst is taken out of a reactor system for regeneration, the catalyst is burnt in air or in oxygen-containing gas. In case of liquid phase reaction, usual general reactors for liquid reactions for solid catalysts can be used. Since a difference in boiling point between glycerin (290°C) and acrolein and acrylic acid is big, the reaction is effected preferably at relatively lower temperatures so as to distil out acrolein continuously.

The reaction temperature for producing acrolein and acrylic acid by dehydration of glycerin in gas phase is effected preferably at a temperature of 450 °C to 200°C. If the temperature is lower than 200°C, the life of catalyst will be shortened due to polymerization and carbonization of glycerin and of reaction products because the boiling point of glycerin is high. On the contrary, if the temperature exceeds 450°C, the selectivity of acrolein and acrylic acid will be lowered due to increment in parallel reactions and successive reactions. Therefore, more preferable reaction temperature is 250°C to 350°C. The pressure is not limited specially but is preferably lower than 5 atm and more preferably lower than 3 atm. Under higher pressures, gasified glycerin will be re-liquefied and deposition of carbon will be promoted by higher pressure so that the life of catalyst will be shortened.

A feed rate of a material gas is preferably 500 to $10,000 h^{-1}$ in term of the space velocity of GHSV. The selectivity will be lowered if the GHSV becomes lower than $500 h^{-1}$ due to successive reactions. On the contrary, if the GHSV exceeds $10,000 h^{-1}$, the conversion will be lowered.

The reaction temperature of the liquid phase reaction is preferably from 150°C to 350°C. The selectivity will be spoiled under lower temperatures although the conversion is improved. The reaction pressure is not limited specially but the reaction can be carried if necessary under a pressurized conditions of 3 atm to 70 atm.

The material of glycerin is easily available in a form of aqueous solution of glycerin. Concentration of the aqueous solution of glycerin is from 5 % to 90 % by weight and more preferably 10 % to 50 % by weight. Too higher concentration of glycerin will result in such problems as production of glycerin ethers or undesirable reaction between the resulting acrolein or acrylic acid and material glycerin. Still more, the energy which is necessary to gasify glycerin is increase.

Now, the present invention will be explained in much detail with referring several examples, but this invention should not be limited to those described in following examples. In the following Examples and Comparative Examples, % means mole %.

## Examples

Example 1

Cesium salt of tungstophosphoric acid (CsPW) was prepared according to JP-A1-4-139149. Namely, 50 g of tungstophosphoric acid ($H_3[PW_{12}O_{40}] nH_2O$, n = about 30, a product of Nippon Inorganic Colour & Chemical Co., Ltd.) was dissolved in 20 ml of pure water to obtain an aqueous solution of tungstophosphoric acid. In a separate beaker, 7.19 g of cesium nitrate ($CsNO_3$, Kishida Chemical Co., Ltd.) was dissolved in 60 ml of water to obtain an aqueous solution of cesium nitrate. The aqueous solution of cesium nitrate was added under stirring drop-wise by means of a dropping funel to the aqueous solution of tungstophosphoric acid. White slurry was generated at every dripping.

The resulting slurry was treated in a rotary evaporator under vacuum at 60°C to obtain white powder. This powder was then dried at 150°C for 6 hours in an oven at ambient pressure. Then, the resulting powder was fired in air at 250°C for 3 hours by using a Muffle furnace to obtain a catalyst (CsPWO) of cesium salt of tungstophosphoric acid having a composition (proportions in material, compositions hereinafter have the same meaning): $H_{0.5}Cs_{2.5}PW_{12}O_{40}$.

The catalyst was evaluated in a fixed bed reactor operated under ambient pressure by passing material flow through the fixed bed.

The resulting catalyst powder was compacted and then crushed. Crushed particles were sleeved to obtain particles of 9 to 12 mech. 10 cc of the catalyst granules or particles was packed in a SUS reaction tube (diameter of 10 mm).

An aqueous solution of glycerin (concentration of 20 % by weight) was fed to an evaporator at a flow rate of 21 g/hr by a pump so that glycerin was gasified at 300°C. The resulting gasified glycerin was passed through the fixed catalyst bed together with air. The fixed catalyst bed was heated at a temperature of 260°C to 350°C. Feed gas had following composition in mol %: glycerin : oxygen : nitrogen : water = 4.2 : 2.2 : 8.1 : 85.5. GHSV was $2445 h^{-1}$.

Products were condensed in a condenser and quantitative-analyzed by a gas chromatograph (GC-4000, a product of GL Science, DB-WAX column). Proportions of products were corrected in factors from the results of the gas chromatograph to determine absolute amounts of products to calculate the conversion (%) of material (the conversion of glycerin), selectivity of objective substance (the selectivity of acrolein) and the yield of objective substance (the yield of acrolein) from an amount of glycerin fed, an amount of glycerin remained and amounts of the products by following equations:

$$\text{The conversion (\%) of material} = (\text{a mole number of material reacted} / \text{a mole number of material supplied}) \times 100$$

$$\text{The selectivity (\%) of objective substance} = (\text{a mole number of objective substance obtained} / \text{a mole number of material reacted}) \times 100$$

$$\text{The yield (\%) objective substance} = (\text{a mole number of objective substance obtained} / \text{a mole number of material fed}) \times 100$$

Results are summarized in Table 1.

Example 2

Procedure of Example I was repeated except that 5.44 g of rubidium nitrate ($RbNO_3$)(Mitsuwa Chemicals Co., Ltd.) was used instead of cesium nitrate ($CsNO_3$) to prepare a catalyst (RbPW) of rubidium salt of tungstophosphoric acid having a composition: $H_{0.5}Rb_{2.5}PW_{12}O_{40}$.
Reaction and evaluation were also effected under the same condition as Example 1.

Example 3

Procedure of Example I was repeated except that 3.22 g of calcium chloride, dihydrate ($CaCl_2$ $2H_2O$)(Wako Pure Chemical Industries, Ltd.) was used instead of cesium nitrate ($CsNO_3$) to prepare a catalyst (CaPW) of calcium salt of tungstophosphoric acid having a composition: $Ca_{1.5}Rb_{2.5}PW_{12}O_{40}$.
Reaction and evaluation were also effected under the same condition as Example 1.

Example 4

Procedure of Example 1 was repeated except that 5.96 g of ferric nitrate (III) nonahydrate (Fe $(NO_3)_3$ $9H_2O$) (Nihon Kagaku Sangyo Co., Ltd.) was used instead of cesium nitrate ($CsNO_3$) to prepare iron salt of tungstophosphoric acid a catalyst (FePW) of calcium salt of tungstophosphoric acid having a composition: $FePW_{12}O_{40}$.
Reaction and evaluation were also effected under the same condition as Example 1.

Example 5

Procedure of Example I was repeated except that 3.57 g of zirconium oxychloride, 8 hydrates ($ZrOCl_2$ $8H_2O$) (Wako Pure Chemical Industries, Ltd.) was used instead of cesium nitrate ($CsNO_3$) to prepare zirconium salt of tungstophosphoric acid (ZrPW) having a composition: $Zr_{0.75}PW_{12}O_{40}$
Reaction and evaluation were also effected under the same condition as Example 1.

Example 6

Procedure of Example I was repeated except that 6.34 g of lanthanum nitrate ($La(NO_3)_3$ $6H_2O$) (Wako Pure Chemical Industries, Ltd.) was used instead of cesium nitrate ($CsNO_3$) to prepare lanthanum salt of tungstophosphoric acid (LaPW) having a composition: $LaPW_{12}O_{40}$.
Reaction and evaluation were also effected under the same condition as Example 1

Example 7

Procedure of Example 1 was repeated except that 3.53 g of hafnium chloride ($HfCl_4$) (Wako Pure Chemical Industries, Ltd.) was used instead of cesium nitrate ($CsNO_3$) to prepare hafnium salt of tungstophosphoric acid (HfPW) having a composition: $Hf_{0.75}W_{12}O_{40}$

Reaction and evaluation were also effected under the same condition as Example 1.

Example 8

Bismuth salt of tungstophosphoric acid (BiPW) was prepared according to JP-Al-4-139149 and JP-A1-2006-110539. Namely, 50 g of tungstophosphoric acid ($H_3[PW_{12}O_{40}]$ $nH_2O$, n = about 30, a product of Nippon Inorganic Colour & Chemical Co., Ltd.) was dissolved in 20 ml of pure water to obtain an aqueous solution of tungstophosphoric acid. In a separate beaker, to 7.09 g of bismuth nitrate ($Bi(NO_3)_3$, Kishida Chemical Co., Ltd.), 28.3 ml of 60% aqueous solution of nitric acid and 117.6 ml of water were added. The resulting aqueous solution of bismuth nitrate was added under stirring drop-wise by means of a dropping funel to the aqueous solution of tungstophosphoric acid. Yellow white slurry was generated at every dripping.

The resulting slurry was dried by a rotary evaporator under vacuum at 60 °C to obtain white powder. This powder was then dried at 150°C for 6 hours in an oven at ambient pressure. Then, the resulting powder was fired in air at 250° C for 3 hours by using a Muffle furnace to obtain a catalyst (BiPW) of bismuth salt of tungstophosphoric acid having a composition: $BiPW_{12}O_{40}$.

Example 9

In preparation of the cesium salt of heteropoly acid in Example 1, tungstosilicic acid was used instead of tungstophosphoric acid to prepare cesium salt of tungstosilicic acid (CsSiW).

Namely, 50 g of tungstosilicic acid (a product of Nippon Inorganic Colour & Chemical Co., Ltd.) in place of tungstophosphoric acid was dissolved in 20 ml of pure water to obtain an aqueous solution of tungstosilicic acid. In a separate beaker, 7.43 g of cesium nitrate ($CsNO_3$, Kishida Chemical Co., Ltd.) was dissolved in 60 ml of water to obtain an aqueous solution of cesium nitrate. The aqueous solution of cesium nitrate was added under stirring drop-wise by means of a dropping funel to the aqueous solution of tungstosilicic acid. White slurry was generated at every dripping.

The resulting slurry was treated in a rotary evaporator under vacuum at 60°C to obtain white powder. This powder was then dried at 150°C for 6 hours in an oven at ambient pressure. Then, the resulting powder was fired in air at 250°C for 3 hours by using a Muffle furnace to obtain cesium salt of tungstophosphoric acid (CsPW) having a composition: $H_{1.5}C_{S2.5}PW_{12}O_{40}$.

Reaction and evaluation were also effected under the same condition as Example 1.

Comparative Examples 1 to 3

To compare with the salts of heteropolyacid, heteropolyacid alone was used and evaluated.

In Comparative Examples 1 to 3, as heteropoly acid, tungstophosphoric acid ($H_3[PW_{12}O_{40}]$ $nH_2O$, n = about 30), tungstosilicic acid ($H_3[SiW_{12}O_{40}]$ $nH_2O$, n =about 24) and phosphomolybdic acid ($H_3[PM_{12}O_{40}$ $nH_2O$, n =about 30, products of Nippon Inorganic Colour & Chemical Co., Ltd) were used and fired for 3 hours in Muffle furnace at 250°C in air.

Reaction and evaluation were also effected under the same condition as Example 1.

Results of these Examples are summarized in Table 1.

Table 1

| | Catalyst | Reaction temperature °C | Glycerin conversion (%) | Acrolein yield (%) |
|---|---|---|---|---|
| Example 1 | CsPW | 260 | 100 | 92.9 |
| 2 | RbPW | 280 | 100 | 91.2 |
| 3 | CaPW | 350 | 78.6 | 49.8 |
| 4 | FePW | 300 | 99.0 | 70.9 |
| 5 | ZrPW | 350 | 82.5 | 60.6 |
| 6 | LaPW | 300 | 95.0 | 65.6 |
| 7 | HfPW | 350 | 84.6 | 62.1 |
| 8 | BiPW | 320 | 85.7 | 60.9 |
| 9 | CsSiW | 280 | 100 | 93.1 |
| Comparative Example 1 | PW | 320 | 74.0 | 54.8 |

(continued)

|  | Catalyst | Reaction temperature °C | Glycerin conversion (%) | Acrolein yield (%) |
|---|---|---|---|---|
| 2 | SiW | 350 | 73.4 | 50.2 |
| 3 | PMo | 260 | 91.3 | 16.3 |

Example 10

Powder of cesium salt of tungstophosphoric acid ($Cs_{2.5}H_{0.5}PW_{12}O_{40}$) (a product of Nippon Inorganic Colour & Chemical Co., Ltd) was fired in air at 250°C for 3 hours by using a muffle furnace to obtain a catalyst.
The catalyst was evaluated in a fixed bed reactor operated under ambient pressure in a fixed bed. Namely, the resulting catalyst powder was compacted and then crushed. Crushed particles were passed through sieves to obtain particles having a particle size of 9 to 12 mesh. 10 cc of the catalyst granules or particles was packed in a SUS reaction tube (diameter of 20 mm).
An aqueous solution of glycerin (a concentration of 30 % by weight) was fed to an evaporator at a flow rate of 21 g/hr by a pump so that glycerin was gasified at 300°C. The resulting gasified glycerin was passed through the fixed catalyst bed together with air. The fixed catalyst bed was heated at a temperature of 260°C to 350°C. Feed gas had a following composition in mol %: glycerin: oxygen: nitrogen: water = 6.3 : 4.0 : 14.9 : 74.8. GHSV was 2,445 h$^{-1}$.
Products were analyzed and the conversion (%) of material (the conversion of glycerin), the conversion of material (glycerin), the selectivity of target substances (the selectivity of acrolein and the selectivity of acrylic acid) and the yield of target substances (the yield of acrolein and the yield of acrylic acid) were calculated by the same method as Example 1. Result is shown in
Table 2.

Example 11

50 g of cesium salt of tungstophosphoric acid ($Cs_{2.5}H_{0.5}PW_{12}O_{40}$) (a product of Nippon Inorganic Colour & Chemical Co., Ltd) was added with 80 ml of pure water. In a separate beaker, 0.008 g of chloroplatinate hexahydrates ($H_2PtCl_6$ $6H_2O$) (Wako Pure Chemical Industries, Ltd) was dissolved in 0.5 ml of water to obtain a solution which was then added under stirring dropwise to a white solution of the cesium salt of tungstophosphoric acid by using a dropping funnel.
The resulting slurry was treated in a rotary evaporator under vacuum at 60°C to obtain white powder. This powder was then dried at 150°C for 6 hours in an oven at ambient pressure. Then, the resulting powder was fired in air at 250°C for 3 hours by using a muffle furnace to obtain a catalyst (Pt-CsPW) of platinum-added cesium salt of tungstophosphoric acid having a following composition: $Pt_{0.001}H_{0.5}Cs_{2.5}PW_{12}O_{40}$.
Reaction and evaluation were effected under the same condition as Example 10. Result is shown in Table 2.

Example 12

Procedure of Example 11 was repeated except that 0.492 g of iron nitrate nonahydrate ($Fe(NO_3)_3$ $9H_2O$) (NIHON KAGAKU SANGYO CO., LTD.) was used instead of the chloroplatinate hexahydrates ($H_2PtCl_6$ $6H_2O$) to prepare a catalyst of iron-added salt of tungstophosphoric acid (Fe-CsPW) having a composition: $Fe_{0.08}H_{0.26}CS_{2.5}PW_{12}O_{40}$.
Reaction and evaluation were effected under the same condition as Example 10. Result is shown in Table 2.

Example 13

Procedure of Example 11 was repeated except that 0.488 g of chromium nitrate nonahydrate ($Cr(NO_3)_3$ $9H_2O$) (Wako Pure Chemical Industries, Ltd) was used instead of the chloroplatinate hexahydrates ($H_2PtCl_6$ $6H_2O$) to prepare a catalyst of iron-added salt of tungstophosphoric acid (Cr-CsPW) having a composition: $Cr_{0.08}H_{0.26}Cs_{2.5}PW_{12}O_{40}$.
Reaction and evaluation were effected under the same condition as Example 10. Result is shown in Table 2.

Example 14

Procedure of Example 11 was repeated except that 0.095 g of ammonium nitrate ($NH_4NO_3$) (Wako Pure Chemical Industries, Ltd) was used instead of the chloroplatinate hexahydrates ($H_2PtCl_6$ $6H_2O$ to prepare a catalyst of ammonium-added salt of tungstophosphoric acid ($NH_4$-CsPW) having a composition: $NH_{4\,0.08}H_{0.42}Cs_{2.5}PW_{12}O_{40}$.
Reaction and evaluation were effected under the same condition as Example 10. Result is shown in Table 2.

Example 15

Procedure of Example 11 was repeated except that 0.182 g of rubidium nitrate (RbNO$_3$) (Mitsuwa Chemical Co, Ltd) was used instead of the chloroplatinate hexahydrates (H$_2$PtCl$_6$ 6H$_2$O) to prepare a catalyst of rubidium-added salt of tungstophosphoric acid (Rb-CsPW) having a composition: Rb$_{0\cdot08}$H$_{0.42}$Cs$_{2.5}$PW$_{12}$O$_{40}$.
Reaction and evaluation were effected under the same condition as Example 10. Result is shown in Table 2.

Example 16

Procedure of Example 11 was repeated except that 1.751 g of telluric (VI) acid (H$_6$TeO3) (Shinko Chemical Co., Ltd.) was used instead of the chloroplatinate hexahydrates (H$_2$PtCl$_6$ 6H$_2$O) to prepare a catalyst of tellurium-added salt of tungstophosphoric acid (Te-CsPW) having a composition: Te$_{0\cdot5}$H$_{0.5}$C$_{S2.5}$PW$_{12}$O$_{40}$.
Reaction and evaluation were effected under the same condition as Example 10. Result is shown in Table 2. ,

Example 17

Procedure of Example 11 was repeated except that 0.125 g of potassium nitrate (KNO$_3$) (Sigma Aldrich) was used instead of the chloroplatinate hexahydrates (H$_2$PtCl$_6$ 6H$_2$O) to prepare a catalyst of potassium-added salt of tungstophosphoric acid (K-CsPW) having a composition: K$_{0.08}$H$_{0.42}$Cs$_{2.5}$PW$_{12}$O$_{40}$.
Reaction and evaluation were effected under the same condition as Example 10. Result is shown in Table 2.

Example 18

Procedure of Example 11 was repeated except that 0.327 g of ammonium perrhenate (NH$_4$ReO$_4$) (Mitsuwa Chemical Co, Ltd) was used instead, of the chloroplatinate hexahydrates (H$_2$PtCl$_6$ 6H$_2$O) to prepare a catalyst of rhenium-added salt of tungstophosphoric acid (Re-CsPW) having a composition: Re$_{0.08}$H$_{0.5}$C$_{S2.5}$PW$_{12}$O$_{40}$.
Reaction and evaluation were effected under the same condition as Example 10. Result is shown in Table 2.

Table 2

|  | Catalyst | Reaction temperature (°C) | Glycerin conversion (%) | Acrolein yield (%) | Acryic acid yield (%) |
|---|---|---|---|---|---|
| Example 10 | CsPW | 280 | 99.4 | 84.2 | 1.0 |
| 11 | Pt-CsPW | 260 | 100 | 74.0 | 4.6 |
| 12 | Fe-CsPW | 300 | 100 | 65.8 | 11.7 |
| 13 | Cr-CsPW | 300 | 100 | 61.4 | 15.2 |
| 14 | NH$_4$-CsPW | 280 | 99.8 | 82.9 | 1.1 |
| 15 | Rb-CsPW | 280 | 99.9 | 83.2 | 1.0 |
| 16 | Te-CsPW | 280 | 100 | 47.7 | 23.4 |
| 17 | K-CsPW | 280 | 99.9 | 87.1 | 1.1 |
| 18 | Re-CsPW | 280 | 100 | 86.2 | 1.1 |

Then, experiments were carried out by supporting the cesium tungstophosphate on niobia (niobium oxide). A degree of support was 30 % by weight. The degree of support is calculated by following equation:
The degree of support (wt%) = 100 * (weight of cesium tungstophosphate) / (weight of cesium tungstophosphate + weight of support)

Example 19

15 g of cesium salt of tungstophosphoric acid (Cs$_{2.5}$H$_{0.5}$PW$_{12}$O$_{40}$) (a product of Nippon Inorganic Colour & Chemical Co., Ltd) was added with 250 ml of pure water and stirred. Into the resulting white solution of the cesium tungstophosphate, 35 g of support of niobia (Mitsui Mining & Smelting Co., Ltd.) and stirred for 2 hours in ambient temperature. The resulting slurry was dried in a rotary evaporator under vacuum at 60°C to obtain white powder. This powder was then dried at 150°C for 6 hours in an oven at ambient pressure. Then, the resulting powder was fired in air at 250°C for 3 hours by using a muffle furnace.

Reaction and evaluation were effected under the same condition as Example 10. Result is shown in Table 3.

Table 3

| | carrier | Reaction temperature (°C) | Glycerin conversion (%) | Acrolein yield (%) | Acryic acid yield (%) |
|---|---|---|---|---|---|
| Example 19 | $Nb_2O_5$ | 300 | 99.7 | 84.4 | 0.6 |

From the comparison between Examples and Comparative Examples, followings are observed:

(1) In the production of acrolein by dehydration reaction of glycerin, the yield of acrolein can be increased remarkably such as higher than 90%, by using the catalyst according to the present invention, in particular, catalyst compounds in which proton in heteropolyacid such as PW and SiW is replaced at least partially by alkali metal such as Cs or Rb.

(2) When heteropolyacid alone (which is outside the present invention) was used, the yield of acrolein is such poor as lower than 55% even in the highest yield of acrolein for PW (tungstophosphoric acid).

(3) The conversion of glycerin and the yield of acrolein are further increased by adding a salt of at least one element belonging to Group 1 to Group 16 of the Periodic Table of Elements, in particular, salts of K, Re to the cation exchanged compound.

(4) The conversion of glycerin and the yield of acrylic acid are further increased by adding a salt of at least one element belonging to Group 1 to Group 16 of the Periodic Table of Elements, in particular, salts of Pt, Fe, Cr and Te.

(5) Supported catalyst in which the cation exchanged compound is supported on carrier such as niobia show similar conversion of glycerin and similar yield of acrolein even much severer operational conditions to which the cation exchanged compound is subjected.

**Claims**

1. Dehydration catalyst for producing acrolein and acrylic acid by catalytic dehydration reaction of glycerin, comprising mainly a compound in which protons in a heteropolyacid are exchanged at least partially with at least one cation selected from elements belonging to Group 1 to Group 16 of the Periodic Table of Elements.

2. The dehydration catalyst of claim 1 for producing acrolein and acrylic acid by a catalytic dehydration reaction of glycerin, comprising mainly a salt or salts of heteropolyacid, in which protons in said heteropolyacid are exchanged at least partially with at least one cation selected from elements belonging to Group 1 to Group 16 of the Periodic Table of Elements, said a compound is represented by the general formula (1):

$$H_a A_b [X_1 Y c Z_d O_e] \cdot nH_2O \qquad (1)$$

in which
H is hydrogen,
A is at least one cation selected from elements belonging to Group 1 to Group 16 of the Periodic Table of Elements, except H X is P or Si,
Y is at least one element selected from the group comprising W, Mo, Ti, Zr, V, Nb, Ta, Cr, Mn, Fe, Co, Ni, Cu, Zn, Ga, In, Tl, Sn and Pb,
Z is at least one element selected from the group comprising W, Mo, Ti, Zr, V, Nb, Ta, Cr, Mn, Fe, Co, Ni, Cu, Zn, Ga, In, Tl, Sn and Pb, and a, b, c and d satisfying following ranges:

$0 \leq a < 9$
$0 < b \leq 9$
$0 < c \leq 12$ and
$0 \leq d < 12$

e is a number determined by the oxidation numbers of the elements and n is any positive number.

3. The catalyst of claim 1 or 2, in which said cation is at least one alkali metal cation.

**4.** The catalyst of claim 3, in which said alkali metal is cesium.

**5.** The catalyst of any one of claims 1 to 4, in which said compound contains at least one element selected from the group comprising W, Mo and V.

**6.** The catalyst of any one of claims 1 to 5, containing further another salt of at least one element selected from elements belonging to Group 1 to Group 16 of the Periodic Table of Elements, in addition to said compound.

**7.** The supported catalyst of any one of claims 1 to 6, wherein said compound is supported on a carrier.

**8.** The supported catalyst of claim 7, wherein said carrier is titania, silica, zirconia, niobia, magnesia ceria or alumina.

**9.** A process for preparing a catalyst for producing acrolein and acrylic acid by dehydration reaction of glycerin, **characterized by** the steps of adding a solution of at least one metal selected from elements belonging to the Group 1 to Group 16 of the Periodic Table of Elements or onium to a solution of heteropolyacid, and firing the resulting solid mixture.

**10.** The process of claim 9, in which the calcination is carried out under an atmosphere of air, inert gas or a mixture of oxygen and inert gas.

**11.** The process of claim 9 or 10, in which calcination is effected at a temperature of 150 to 900°C for 0.5 to 10 hours.

**12.** Use of the catalyst defined in any one of claims 1 to 6 or of the supported catalyst of claim 7 or 8 in production of acrolein or acrylic acid by catalytic dehydration reaction of glycerin.

<p style="text-align:center">**INTERNATIONAL SEARCH REPORT**</p>

| International application No. |
|---|
| PCT/JP2009/057819 |

**A. CLASSIFICATION OF SUBJECT MATTER**
B01J27/188(2006.01)i, B01J23/30(2006.01)i, B01J37/08(2006.01)i, C07C27/00
(2006.01)i, C07C45/52(2006.01)i, C07C47/22(2006.01)i, C07C51/00(2006.01)i,
C07C51/235(2006.01)i, C07C57/04(2006.01)i, C07B61/00(2006.01)n
According to International Patent Classification (IPC) or to both national classification and IPC

**B. FIELDS SEARCHED**

Minimum documentation searched (classification system followed by classification symbols)
B01J27/188, B01J23/30, B01J37/08, C07C27/00, C07C45/52, C07C47/22,
C07C51/00, C07C51/235, C07C57/04, C07B61/00

Documentation searched other than minimum documentation to the extent that such documents are included in the fields searched
Jitsuyo Shinan Koho        1922–1996   Jitsuyo Shinan Toroku Koho   1996–2009
Kokai Jitsuyo Shinan Koho  1971–2009   Toroku Jitsuyo Shinan Koho   1994–2009

Electronic data base consulted during the international search (name of data base and, where practicable, search terms used)
Science Direct, WPI, JSTPlus(JDreamII), JST7580(JDreamII)

**C. DOCUMENTS CONSIDERED TO BE RELEVANT**

| Category* | Citation of document, with indication, where appropriate, of the relevant passages | Relevant to claim No. |
|---|---|---|
| A | JP 2005-131470 A  (Nippon Shokubai Co., Ltd.), 26 May, 2005 (26.05.05), The entire specification (Family: none) | 1-12 |
| A | WO 2006/087084 A2  (ARKEMA FRANCE), 24 August, 2006 (24.08.06), Claim 4 & US 2008/0214880 A1    & EP 1848681 A & DE 602006001638 D    & FR 2882053 A & FR 2882053 A1    & KR 10-2007-0104413 A & CN 101119956 A    & AT 399751 T & ES 2308717 T | 1-12 |

☒ Further documents are listed in the continuation of Box C.      ☐ See patent family annex.

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 07 September, 2009 (07.09.09) | 15 September, 2009 (15.09.09) |

| Name and mailing address of the ISA/ Japanese Patent Office | Authorized officer |
|---|---|
| Facsimile No. | Telephone No. |

Form PCT/ISA/210 (second sheet) (April 2007)

**INTERNATIONAL SEARCH REPORT**

International application No.

PCT/JP2009/057819

C (Continuation).   DOCUMENTS CONSIDERED TO BE RELEVANT

| Category* | Citation of document, with indication, where appropriate, of the relevant passages | Relevant to claim No. |
|---|---|---|
| A | WO 2007/058221 A1  (Nippon Shokubai Co., Ltd.), 24 May, 2007 (24.05.07), The entire specification & JP 2007-137785 A      & JP 2008-88149 A | 1-12 |

Form PCT/ISA/210 (continuation of second sheet) (April 2007)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2558520 A
- JP 2006290815 A
- JP 6211724 A
- WO 2007058221 A
- WO 2006087083 A
- WO 2006087084 A
- JP 4139149 A
- JP 4139149 A1
- JP 2006110539 A

**Non-patent literature cited in the description**

- **Tsukuda et al.** Production of acrolein from glycerol over silica-supported heteropoly acids. *CATALYSIS COMMUNICATIONS,* 21 July 2007, vol. 8 (9), 1349-1353